# EUROPÄISCHE PATENTSCHRIFT

(11) **EP 4 117 748 B1**
(45) Veröffentlichungstag und Bekanntmachung des Hinweises auf die Patenterteilung: **09.10.2024**
(21) Anmeldenummer: 21707923.5
(22) Anmeldetag: 22.02.2021
(51) Int. Cl.: A61M 1/16

(54) **REINIGUNGSVORRICHTUNG UND REINIGUNGSSYSTEM FÜR EINE BLUTBEHANDLUNGSVORRICHTUNG**
CLEANING DEVICE AND SYSTEM FOR A BLOOD TREATMENT SYSTEM
DISPOSITIF ET SYSTÈME DE NETTOYAGE POUR UN SYSTÈME DE TRAITEMENT SANGUIN

(30) Priorität: 11.03.2020 DE 102020106593
(43) Veröffentlichungstag der Anmeldung: 18.01.2023
(73) Patentinhaber: Fresenius Medical Care Deutschland GmbH, 61352 Bad Homburg (DE)
(72) Erfinder: BREHM, Winfried, 97461 Hofheim (DE); STERZER, Rafael, 97422 Schweinfurt (DE); SEIT, Paul, 97421 Schweinfurt (DE)
(74) Vertreter: Wagner, Andrea
(86) Internationale Anmeldenummer: PCT/EP2021/054281
(87) Internationale Veröffentlichungsnummer: WO 2021/180451

(56) Entgegenhaltungen:
- EP-A1- 1 344 550
- EP-A1- 2 682 608
- EP-A2- 0 352 540
- WO-A2-2013/135389
- DE-T2- 60 207 683

## Beschreibung

### Technisches Gebiet

Die vorliegende Erfindung betrifft eine Reinigungsvorrichtung, ein Reinigungssystem sowie eine Verwendung dieser bei einer Blutbehandlungsvorrichtung, insbesondere eine Dialysemaschine.

### Hintergrund

Unter dem Begriff der Blutbehandlungsvorrichtung ist unter anderem eine Dialysemaschine zu verstehen. Dialysemaschinen finden häufig in Dialysezentren zur Behandlung einer chronischen Nierenerkrankung Anwendung.

Während der Dialyse strömt durch die Blutkammer des Dialysators kontinuierlich Blut des Patienten, während durch die Dialysierflüssigkeitskammer fortlaufend Dialysierflüssigkeit fließt. Zur Herstellung von Dialysierflüssigkeit können vorgefertigte Dialysierflüssigkeitskonzentrate verwendet werden, die in den Dialysevorrichtungen mit Wasser verdünnt werden. In Dialysezentren werden Dialysierflüssigkeitskonzentrate entweder als vorgefertigtes Produkt in Kanistern, Beuteln oder Kartuschen zur Verfügung gestellt oder über ein Ringleitungssystem aus einem zentralen Tank bereitgestellt.

Zentral zur Verfügung gestellte Dialysierflüssigkeitskonzentrate sind für den Anwender einfach zu handhaben, sie haben aber den Nachteil, dass die Dialysierflüssigkeit nicht individuell auf die Bedürfnisse des Patienten abgestimmt werden kann. Dezentral bereitgestellte Konzentrate erlauben zwar eine individuelle Anpassung der Dialysierflüssigkeit an den Patienten, sie müssen aber für jede einzelne Dialysebehandlung an die Dialysevorrichtung gebracht werden.

In der Dialyse finden für die Herstellung eines flüssigen Dialysekonzentrats Beutel oder Kartuschen Verwendung, die mit einem pulverförmigen Dialysierflüssigkeitskonzentrat befüllt sind. Die Konzentratbeutel oder -kartuschen enthalten eine Menge an pulverförmigen Dialysierflüssigkeitskonzentrat, die für eine einzige Dialysebehandlung ausreichend ist. Die Beutel oder Kartuschen sind mit Bikarbonat befüllt. Handelsübliche Bikarbonatbeutel enthalten 650 bis 950 g Natriumbicarbonat. Aus dem pulverförmigen Bikarbonat-Konzentrat wird zunächst ein flüssiges Bikarbonat-Konzentrat hergestellt. Für die Herstellung der Dialysierflüssigkeit ist ein weiteres Säurekonzentrat erforderlich, das in einem Kanister oder mit einer zentralen Versorgung bereitgestellt wird. Bikarbonat-Konzentrat und Säurekonzentrat werden dann mit Wasser zu der fertigen Dialysierflüssigkeit gemischt.

Die Dialysemaschine weist daher ferner einen Wasseranschluss auf, über welchen Reinwasser zugeführt wird. In der Dialysemaschine erfolgt anschließend die gewünschte Mischung von Reinwasser mit dem entsprechenden Konzentrat.

Die Bereitstellung des flüssigen Konzentrat über Konzentratbehälter bringt verschiedene Herausforderungen mit sich, da die Konzentratbehälter schwer und damit für das Pflegepersonal aufwendig in der Handhabung sind. Zudem müssen zur Bereitstellung des Konzentrats Ansaugstäbe der Dialysemaschine in die Konzentratbehälter eingebracht werden, welche nach einer Behandlung zur Reinigung wieder in die Dialysemaschine gesteckt werden. Diese Handhabung der Konzentratbehälter ist damit zeitintensiv und kann zu einer Verlängerung der Pausen zwischen den einzelnen Behandlungen führen.

Alternativ kann das Konzentrat auch über eine Zentralversorgung und einen entsprechenden Zentralversorgungsanschluss an der Dialysemaschine zugeführt werden. Dies setzt aber eine entsprechende Ausstattung der Klinik voraus, und kann bezüglich der Flexibilität des zur Verfügung stehenden Konzentrats und dem Wartungsaufwand der hierfür betriebenen Leitungen Nachteile mit sich bringen.

Aus der EP 1 344 550 A1 ist ein Konnektor zur Verbindung eines Konzentratbeutels, welcher mit Trockenkonzentrat gefüllt ist und an einer Dialysemaschine eingehängt werden kann, bekannt. Auch bei dieser Art der Konzentratversorgung sind bestimmte Schritte zwischen den Behandlungen notwendig. So muss ein am Konzentratbeutel angebrachter Kontaminationsschutz manuell entfernt, der Konzentratbeutel in die entsprechende Aufnahme an der Dialysemaschine eingesetzt, das heißt konnektiert werden, und schließlich mit dem Dialysatkreislauf verbunden werden.

Die Konnektion des Konzentratbeutels erfolgt im Stand der Technik zumeist von unten. Das heißt der Konzentratbeutel wird auf Konnektionstüllen aufgesteckt. Nach der Behandlung wird der Konzentratbeutel von den Konnektionstüllen gehoben. Dabei kann Restflüssigkeit, welche noch in den Anschlussstellen des Konzentratbeutels vorhanden ist, leicht auf den Boden tropfen.

Dabei kann Restflüssigkeit der Bicarbonatlösung aber auch zu Ablagerungen an den Konnektionstüllen der Dialysemaschine führen. Dies wird durch Kalzium- und Magnesiumsalze verursacht. Durch die Ausbildung der nach unten geöffneten Anschlussstellen kann diese Restflüssigkeit in die Anschlusstüllen tropfen und dort die Ablagerungen verursachen. Diese können zu Beschädigungen von den Anschlussstellen zugeordneten Dichtringen führen.

Nach einer Behandlung muss der Konzentratbeutel von dem Dialysatkreislauf dekonnektiert und entfernt werden. Erst nach der Entfernung kann ein Reinigungsprogramm an der Dialysemaschine gestartet werden. Auch die Oberflächenreinigung der Dialysemaschine kann erst nach dem Entfernen des Konzentratbeutels von dem Pflegepersonal durchgeführt werden.

Neben hohen Sicherheitsanforderungen spielt bei der Dialysebehandlung, insbesondere in Dialysezentren, der zeitliche Aspekt eine besondere Rolle.

Da ausreichend Zeit für die tatsächliche Behandlung zur Verfügung stehen soll, ist insbesondere die Zeit zwischen den Behandlungen einer ständigen Zeitoptimierung unterworfen. Wie oben beschrieben, nimmt zwischen den Behandlungen neben der Vorbereitung des Patienten, auch die Vorbereitung der Dialysemaschine für die nächste Behandlung, durch Schritte, wie der Bereitstellung der benötigten Konzentratbehälter oder der Desinfizierung der Dialysemaschine, Zeit in Anspruch.

Der vorliegenden Anmeldung liegt damit die Aufgabe zu Grunde eine Reinigungsvorrichtung für eine Blutbehandlungsvorrichtung bereit zu stellen, die eine verbesserte Handhabung und/oder Zeitersparnis erlaubt. Zudem soll die Sicherheit des Patienten durch eine einfachere Reinigung der Blutbehandlungsvorrichtung gewährleitest werden.

### Zusammenfassung der Erfindung

Die der Erfindung zugrunde liegende Aufgabe wird durch die Reinigungsvorrichtung für eine Blutbehandlungsvorrichtung gemäß Anspruch 1 sowie durch das Reinigungssystem für eine Blutbehandlungsvorrichtung nach Anspruch 10 gelöst.

Vorteilhafte Weiterentwicklungen und Ausführungsformen sind Gegenstand der abhängigen Ansprüche.

Erfindungsgemäß ist eine Reinigungsvorrichtung für eine Blutbehandlungsvorrichtung vorgesehen, welche einen Grundkörper, ein erstes und ein zweites Leitungselement, welche sich in dem Grundkörper von einer ersten Oberfläche des Grundkörpers, vorzugsweise senkrecht, erstrecken, vorgesehen. Weiter kann die Reinigungsvorrichtung ein erstes Durchgangsloch aufweisen. Das erste Durchgangsloch kann sich von an einem von der ersten Oberfläche des Grundkörpers beabstandeten ersten Stelle des ersten Leitungselements zu einer von der ersten Oberfläche des Grundkörpers verschiedenen Oberfläche des Grundkörpers erstrecken. Damit kann das erste Durchgangsloch eine fluidische Verbindung von dem ersten Leitungselement zur einer Außenseite der Reinigungsvorrichtung erstrecken. Ebenso kann sich das Durchgangsloch zu einer Innenseite des zweiten Leitungselements erstrecken.

Weiter kann die Reinigungsvorrichtung ein erstes Anschlusselement aufweisen, welches sich von dem Grundkörper, vorzugsweise entlang der Mittellinie, des ersten Durchgangslochs von der ersten Seitenfläche des Grundkörpers erstreckt, so dass sich das Durchgangsloch von der ersten Seitenfläche des Grundkörpers weiter durch das erste Anschlusselement erstreckt. Das Anschlusselement kann dabei integral mit der Reinigungsvorrichtung ausgebildet sein oder in dieses eingepresst, verklebt oder verschraubt werden. Das Anschlusselement kann dabei aus einem anderen Material ausgebildet sein. Beispielsweise kann der Grundkörper aus Metall, und das Anschlusselement aus einem flexiblen Material, beispielsweise Kunststoff ausgebildet werden. Das erste Anschlusselement kann dabei ein sich von dem Grundkörper, vorzugsweise im Wesentlichen senkrecht, erstreckender, vorzugsweise zylinderförmig, konisch oder stufenförmig ausgebildeter, Vorsprung sein.

Erfindungsgemäß ist eine Reinigungsvorrichtung für eine Blutbehandlungsvorrichtung eine Vorrichtung, welche zur Reinigung der Blutbehandlungsvorrichtung, insbesondere einer Dialysemaschine eingesetzt werden kann. Insbesondere können durch die Reinigungsvorrichtung die zur Konnektion eines Konzentratbehälters mit der Dialysemaschine vorgesehenen Anschlusstüllen gereinigt werden. Da die Anschlusstüllen von oben in die Reinigungsvorrichtung eingebracht werden kann, ist es möglich ein Tropfen von Reinigungsflüssigkeit auf den Boden und insbesondere in die Anschlusstüllen zu verhindern.

Das in dem Grundkörper der Reinigungsvorrichtung angeordnete erste Durchgangsloch erstreckt sich an einer ersten Stelle aus dem ersten Leitungselement, welche von der Oberfläche entfernt ist. Die Leitungselemente sind beispielsweise als Sacklöcher oder Sackbohrungen ausgebildet. Insbesondere kann sich das Durchgangsloch derart an von der Innenseite des ersten Leitungselements erstrecken, so dass Flüssigkeit in dem ersten Leitungselement durch das Durchgangsloch leicht hinein und somit aus dem Leitungselement leicht ausströmen kann. Mit anderen Worten kann das Durchgangsloch derart ausgebildet sein, dass sich dieses von der Unterseite des Leitungselements wegerstreckt.

Nach einer Weiterbildung der Reinigungsvorrichtung kann ein Verbindungskanal zwischen dem ersten und dem zweiten Leitungselement an einer von der Oberfläche des Grundkörpers entfernten zweiten Stelle der Leitungselemente ausgebildet sein. Die Reinigungsvorrichtung kann zudem von den Leitungselementen verschiedene Ausnehmungen aufweisen. Diese Ausnehmungen können eine fluidische Verbindung zwischen einem an der Reinigungsvorrichtung angebrachten Konzentratbeutel und Konnektionstüllen einer Dialysemaschine zulassen.

Der Verbindungskanal kann damit eine fluidische Verbindung zwischen den Leitungselementen herstellen. Der Verbindungskanal kann nahe an einer Bodenfläche der Leitungselemente ausgebildet sein oder erstreckt sich auf einer Ebene mit seiner Unterseite von der Bodenfläche weg. Der Verbindungskanal kann sich im Wesentlichen senkrecht zu der Ausrichtung des Durchgangslochs erstrecken.

Nach einer Weiterbildung der Reinigungsvorrichtung kann sich das erste Leitungselement zu einer ersten Seitenfläche des Grundkörpers erstrecken. Weiter kann das zweite Leitungselement an einer von der ersten Oberfläche des Grundkörpers entfernten dritten Stelle ein zweites Durchgangsloch zu der von der ersten Oberfläche des Grundkörpers verschiedenen ersten Seitenfläche des Grundkörpers aufweisen.

Dabei kann das zweite Durchgangsloch parallel zu dem ersten Durchgangsloch in dem Grundkörper ausgebildet sein. Das zweite Durchgangsloch kann dabei entsprechend dem ersten Durchgangsloch eine Durchgangsbohrung sein, welche eine Außenseite, die auch der Unterseite des Grundkörpers entsprechen kann, mit dem zweiten Leitungselement verbinden. Durch das zweite Durchgangsloch kann insbesondere Fluid aus dem zweiten Leitungselement ausströmen. Das zweite Durchgangsloch ist damit auf einer Ebene mit der Unterseite des zweiten Leitungselements ausgebildet oder nahe an der Unterseite ausgebildet. Das erste und zweite Durchgangsloch können sich aber auch von der Verbindungsstelle mit dem ersten und zweiten Leitungselement von der Oberfläche des Grundkörpers weg abfallen, so dass Fluid leicht aus dem ersten und zweiten Leitungselement herausströmen kann.

Nach einer Weiterbildung der Reinigungsvorrichtung kann in dem ersten und zweiten Leitungselement zumindest ein Sperrelement angebracht sein. Damit kann eine fluidische Verbindung zwischen dem Verbindungskanal mit dem ersten und zweiten Durchgangsloch verhindert werden. Das Sperrelement kann zudem Sollbruchstellen, beispielsweise durch eine verminderte Wandstärke in einem Teilbereich aufweisen.

Das Sperrelement kann ein kreisflächiges Kunststoffelement sein, welches jeweils am Innenumfang der Leitungselemente ausgebildet ist. Zudem kann das Sperrelement selbst Sollbruchstellen aufweisen, welche bei einem Einfahren von Konnektionstüllen in die Leitungselemente das Sperrelement durchstoßen, so dass eine fluidische Verbindung zwischen den Konnektionstüllen und den Durchgangslöchern ausgebildet werden kann. Alternativ oder zusätzlich kann das Sperrelement auch jeweils Sollbruchstellen in der Befestigung am Innenumfang haben. Die Sollbruchstellen könne perforierte bogenförmige Abschnitte des Sperrelements sein, so dass an diesen Stellen die Wandstärke der Sperrelemente dünner ausgebildet ist. Alternativ kann das Sperrelement in dem Verbindungskanal zwischen den Leitungselementen ausgebildet sein.

Nach einer Weiterbildung der Reinigungsvorrichtung kann der Grundkörper aus einem ersten Grundelement und einem zweiten Grundelement ausgebildet sein. Dabei kann jeweils das erste und zweite Grundelement eine im Querschnitt halbkreisförmige Rille zur Ausbildung des Verbindungskanals aufweisen, und das erste und zweite Grundelement können jeweils zwei im Querschnitt halbkreisförmige Aussparungen aufweisen zur Ausbildung von jeweils dem ersten und zweiten Leitungselement bei Verbindung des ersten und zweiten Grundelements.

Dabei können das erste und zweite Grundelement zur Ausbildung des Verbindungskanals und des Leitungselements miteinander fest oder wieder trennbar miteinander verbunden, mit anderen Worten verschraubt, verklebt oder verschweißt sein. Um die Leitungselemente sowie den Verbindungskanal fluidisch dicht auszubilden, kann eine Dichtkontur zwischen dem ersten und zweiten Grundelement ausgebildet sein.

Nach einer Weiterbildung der Reinigungsvorrichtung kann der Grundkörper aus einem ersten Grundelement und einem zweiten Grundelement ausgebildet sein, und das erste Grundelement kann zwei Sackbohrungen und das zweite Grundelement zwei den im Durchmesser der Sackbohrungen entsprechende Durchgangsbohrungen zur Ausbildung des ersten und zweiten Leitungselements aufweisen, zudem können das erste Grundelement und zweite Grundelement eine im Querschnitt halbkreisförmige Rille zur Ausbildung des Verbindungskanals aufweisen.

Nach einer Weiterbildung der Reinigungsvorrichtung kann diese weiter eine erste zylinderförmige Aufnahme mit zumindest einer, vorzugsweise zwei, Durchgangsöffnungen und eine zweite zylinderförmige Aufnahme mit einer Durchgangsöffnung aufweisen. Zudem kann an den Durchgangsöffnungen jeweils ein zylinderförmiger Fortsatz vorgesehen sein, und zumindest ein, vorzugsweise zwei, röhrenförmige Verbindungselemente aufweisen, welche auf den Fortsätzen anbringbar sind und in den Verbindungskanal und/oder das Durchgangsloch derart eingebracht werden können, dass sie die in den Leitungselementen aufgenommenen Aufnahmen verbinden. Zudem können das erste Durchgangsloch und/oder der Verbindungskanal zur ersten Oberfläche geöffnet sein, so dass jeweils die zylinderförmigen Aufnahmen sowie die röhrenförmigen Verbindungselemente in den Grundkörper einlegbar sind.

Nach einer Weiterbildung der Reinigungsvorrichtung können das erste und zweite Leitungselement in ihrer Mittellinie zueinander parallel verlaufen und/oder das erste und das zweite Durchgangsloch können in ihrer Mittellinie zueinander parallel verlaufen.

Nach einer Weiterbildung der Reinigungsvorrichtung kann ein zwischen dem ersten und zweiten Leitungselement ein Mittelelement ausgebildet sein. Das Mittelelement kann dabei bogenförmig ausgebildet sein, und sich von der ersten Oberfläche, vorzugsweise senkrecht, wegerstreckt. Ein Mittelabschnitt zwischen dem ersten und zweiten Leitungselement kann damit offen ausgebildet sein.

Nach einer Weiterbildung der Reinigungsvorrichtung kann zumindest eine Dichtschale in dem ersten und/oder zweiten Leitungselement ausgebildet sein, wobei die Dichtschale zylinderförmig ist, und zumindest eine Seitendurchgangsöffnung aufweisen kann, die in ihrem Durchmesser dem Verbindungskanal und/oder dem Durchgangsloch entspricht, und an der Mantelfläche der Dichtschale ausgebildet sein kann.

Erfindungsgemäß ist ein Reinigungssystem für eine Blutbehandlungsvorrichtung, insbesondere eine Dialysemaschine, ausgebildet, und kann ein Gehäuse zur Anbindung an eine Blutbehandlungsvorrichtung, insbesondere eine Dialysemaschine, eine Reinigungsvorrichtung, vorzugsweise nach einem der vorhergehenden Aspekte aufweisen, die in dem Gehäuse bewegbar angeordnet ist. Zudem kann ein erstes Verschlusselement, mit welchem eine Fluidströmung aus dem ersten Leitungselement über das erste Anschlusselement verhinderbar ist, und/oder ein zweites Verschlusselement mit welchem eine Fluidströmung aus dem zweiten Leitungselement über das zweite Anschlusselement verhinderbar ist ausgebildet sein. Zudem kann ein Antriebselement zum Bewegen der Reinigungsvorrichtung ausgebildet sein. Das Antriebselement kann dabei im Gehäuse oder außerhalb des Gehäuses angeordnet sein. Das Antriebselement kann ein Elektromotor sein, oder ein hydraulisch oder pneumatischer Antrieb sein. Zudem kann, vorzugsweise in dem Gehäuse, ein Sensor zum Erfassen einer Bewegung der Reinigungsvorrichtung angeordnet sein.

Nach einer Weiterbildung des Reinigungssystems kann der Sensor eine Lichtschranke oder ein Hall-Sensor sein. Die Lichtschranke kann auf einer, vorzugsweise oberhalb des Reinigungsvorrichtung angebrachten, Platine sein.

Nach einer Weiterbildung des Reinigungssystems kann zumindest eine in dem Gehäuse, vorzugsweise integral, ausgebildete Führungsschiene, auf welcher die Reinigungsvorrichtung verschiebbar ist, aufweisen. Das erste Antriebselement kann weiter eine Spindelmutter und einen Elektromotor mit einer Gewindespindel aufweisen.

Nach einer Weiterbildung des Reinigungssystems kann die Gewindemutter an dem, vorzugsweise bogenförmig ausgebildeten, Mittelelement der Reinigungsvorrichtung ausgebildet sein und ein anderes Material als der Grundkörper aufweisen, oder das, vorzugsweise bogenförmig ausgebildete, Mittelelement kann einen in Richtung der Lichtschranke ausgebildeten Vorsprung aufweisen, so dass bei einem Bewegen, insbesondere einem Verschieben, der Reinigungsvorrichtung ein Auslösen der Lichtschranke erreichbar ist.

Nach einer Weiterbildung ist eine Verwendung eines Reinigungssystems nach einem vorhergehenden Aspekte bei einer Blutbehandlungsvorrichtung, insbesondere einer Dialysemaschine, vorgesehen.

Nach einer Weiterbildung ist eine Verwendung einer Reinigungsvorrichtung nach einem der vorhergehenden Aspekte bei einer Blutbehandlungsvorrichtung, insbesondere einer Dialysemaschine, vorgesehen.

Durch die Reinigungsvorrichtung sowie Reinigungssystem ist es möglich einen Konzentratbeutel an der Blutbehandlungsvorrichtung anzubringen, während noch ein Reinigungsschritt der Konnektionstüllen erfolgt. Durch die Ausbildung der Reinigungsvorrichtung ist es zudem möglich, die Sicherheit des Patienten zu gewährleisten. Insbesondere kann ein Heraustropfen von Flüssigkeit aus einem mit der Blutbehandlungsvorrichtung, insbesondere Dialysemaschine, verbundenen Konzentratbeutel, verhindert werden.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. In den Figuren sind gleiche Merkmale/Elemente und Merkmale/Elemente mit der gleichen Funktion mit den gleichen Bezugszeichen gekennzeichnet.

### Kurzbeschreibung der Zeichnungen

Hierbei zeigt:
- Fig. 1: eine Prinzipdarstellung einer Reinigungsvorrichtung nach einer ersten Ausführungsform;
- Fig. 2a, 2b: eine schematische Verwendung der Reinigungsvorrichtung nach der ersten Ausführungsform;
- Fig. 3: eine Prinzipdarstellung der Reinigungsvorrichtung mit einem Sperrelement;
- Fig. 4: eine Prinzipdarstellung der Reinigungsvorrichtung nach einer zweiten Ausführungsform;
- Fig. 5: eine Prinzipdarstellung der Reinigungsvorrichtung nach einer dritten Ausführungsform;
- Fig. 6: eine Prinzipdarstellung der Reinigungsvorrichtung nach einer vierten Ausführungsform;
- Fig. 7: eine Prinzipdarstellung der Reinigungsvorrichtung nach einer fünften Ausführungsform;
- Fig. 8: eine Prinzipdarstellung einer Reinigungsschale für eine Reinigungsvorrichtung;
- Fig. 9a: eine schematische Darstellung einer Reinigungsvorrichtung nach einer sechsten Ausführungsform;
- Fig. 9b: eine Prinzipdarstellung der Reinigungsvorrichtung nach der sechsten Ausführungsform zur Verwendung in einer Blutbehandlungsvorrichtung,
- Fig. 10: eine Prinzipdarstellung einer Reinigungsvorrichtung nach einer siebten Ausführungsform zur Verwendung in einer Blutbehandlungsvorrichtung;
- Fig. 11a,: b eine Prinzipdarstellung der Reinigungsvorrichtung nach der siebten Ausführungsform zur Verwendung in einer Blutbehandlungsvorrichtung;
- Fig. 12a,: b eine Prinzipdarstellung der Reinigungsvorrichtung nach einer achten Ausführungsform;
- Fig. 13a, b: Prinzipdarstellung einer Verwendung der Reinigungsvorrichtung nach der achten Ausführungsform;
- Fig. 14: eine Prinzipdarstellung einer Reinigungsvorrichtung nach einer neunten Ausführungsform;
- Fig. 15: eine Prinzipdarstellung eines Reinigungssystems,
- Fig. 16: einen Gehäusedeckel und einen Gehäuseausschnitt, und
- Fig. 17: eine Prinzipdarstellung von Konnektionstüllen zum Einbringen in eine Reinigungsvorrichtung.

### Detaillierte Beschreibung eines Ausführungsbeispiels

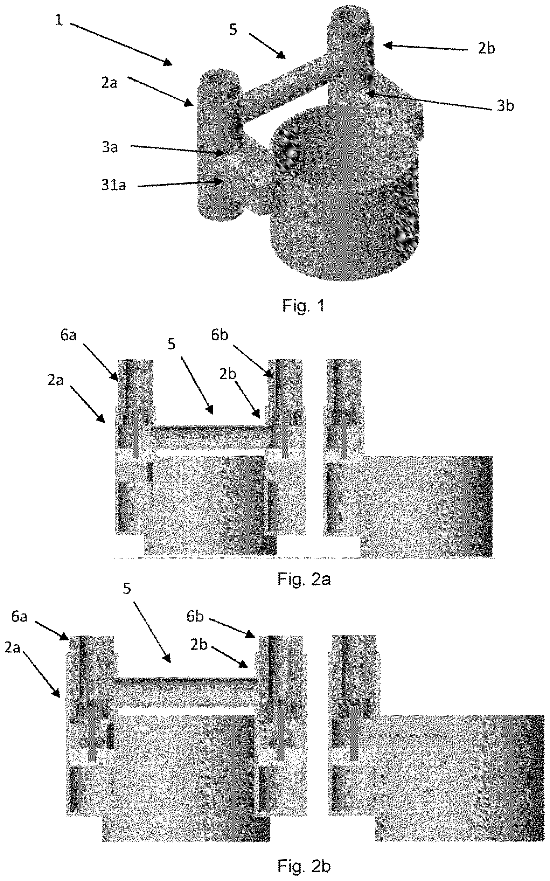

Bezugnehmend auf Fig. 1 wird nachstehend eine erste Ausführungsform erläutert werden. Dabei zeigt Fig. 1 eine Reinigungsvorrichtung mit einem Grundkörper 1. Der Grundkörper 1 weist weiter ein erstes und zweites Leitungselement 2a, 2b auf, welche hier als Hohlzylinder ausgebildet sind und an einer Unterseite des ersten und zweiten Leitungselements 2a, 2b jeweils eine Bodenplatte aufweisen und damit an einer Unterseite fluidisch verschlossen sind. Jedes der zwei Leitungselemente 2a, 2b weist ein Durchgangsloch 3a, 3b auf, das an einer von der Oberflächenseite verschiedenen Seite an den Leitungselementen 2a, 2b ausgebildet ist. Die Leitungselemente 2a, 2b sind hier zueinander parallel verlaufend ausgebildet.

Weiter sind die Durchganglöcher 3a, 3b in einem zu der Oberfläche gleichen Abstand ausgebildet. Von den Durchgangslöchern 3a, 3b erstrecken sich jeweils parallel zueinander die Anschlusselemente 31a, 31b. In der hier in Fig. 1 dargestellten Ausführungsform münden die Anschlusselemente 31a, 31b in einem, zwischen den Leitungselementen 2a, 2b liegenden Adapterhohlzylinder. An diesem Adapterhohlzylinder ist ein Beutel mit Bicarbonatkonzentrat befestigbar. Der Konzentratbeutel kann ein flexibles Material, insbesondere aus PVC, PVT oder Polyethylen, aufweisen und mit dem Grundkörper 1 verschweißt werden. Zwischen den Leitungselementen 2a, 2b verläuft oberhalb der Anschlusselemente 31a, 31b, das heißt näher an der Oberfläche des Grundkörpers 1, ein Verbindungskanal 5. Der Verbindungskanal 5 verbindet die Leitungselemente 2a, 2b fluidisch, so dass ein von der Oberflächenseite einströmendes Fluid über das erste Leitungselement 2a und den Verbindungskanal 5 zu dem zweiten Leitungselement 2a, 2b und von dort aus dem Grundkörper 1 ausströmen kann.

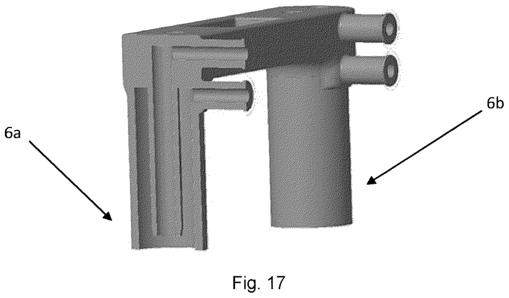

Wird diese Reinigungsvorrichtung bei einer Blutbehandlung, insbesondere einer Dialysebehandlung verwendet, so kann in dieser Reinigungsvorrichtung ein Reinigungsschritt erfolgen. Hierfür werden wie in Fig. 2a gezeigt, in die Leitungselemente 2a, 2b von der Oberfläche des Grundkörpers 1 Anschlusstüllen 6a, 6b, wie in Fig. 17 gezeigt, welche fluidisch mit der Dialysemaschine verbunden sind, eingefahren. Hierfür können an den Innenflächen der Leitungselemente 2a, 2b Linearführungen vorgesehen sein, in welchen die Anschlusstüllen 6a, 6b linear verschiebbar verfahren werden können. Alternativ können die Innenflächen glatt ausgebildet sein, so dass sich die Anschlusstüllen 6a, 6b linear verschieben lassen. Alternativ können Innengewinde in den Leitungselementen angeordnet sein, so dass die Anschlusstüllen 6a, 6b mit einer Drehbewegung eingebracht werden.

Der Arbeitsablauf zur Reinigung der Konnektionstüllen mittels der Reinigungsvorrichtung beginnt mit einem Anbringen der Reinigungsvorrichtung, an welcher ein Konzentratbeutel angebracht ist, an einer Dialysemaschine. Die Anschlusstüllen 6a, 6b der Dialysemaschine verfahren in die Leitungselemente 2a, 2b. Dabei ist an den Anschlusstüllen 6a, 6b eine in Fig. 2 gezeigte Stempelvorrichtung angebracht. Verfahren die Konnektionstüllen in die Leitungselemente 2a, 2b in einer ersten, oberen Position, wie in Fig. 2 gezeigt, verschließen die Stempelelemente die Leitungselemente 2a, 2b unterhalb. Mit anderen Worten befindet sich die Oberseite der Stempelelemente in einer Ebene mit der Unterseite des Verbindungskanals 5. Damit kann Fluid, aus den Anschlusstüllen 6a, 6b ausströmend, nicht zu den Durchgangslöchern 3a, 3b, oder Anschlusselementen 31a, 31b strömen.

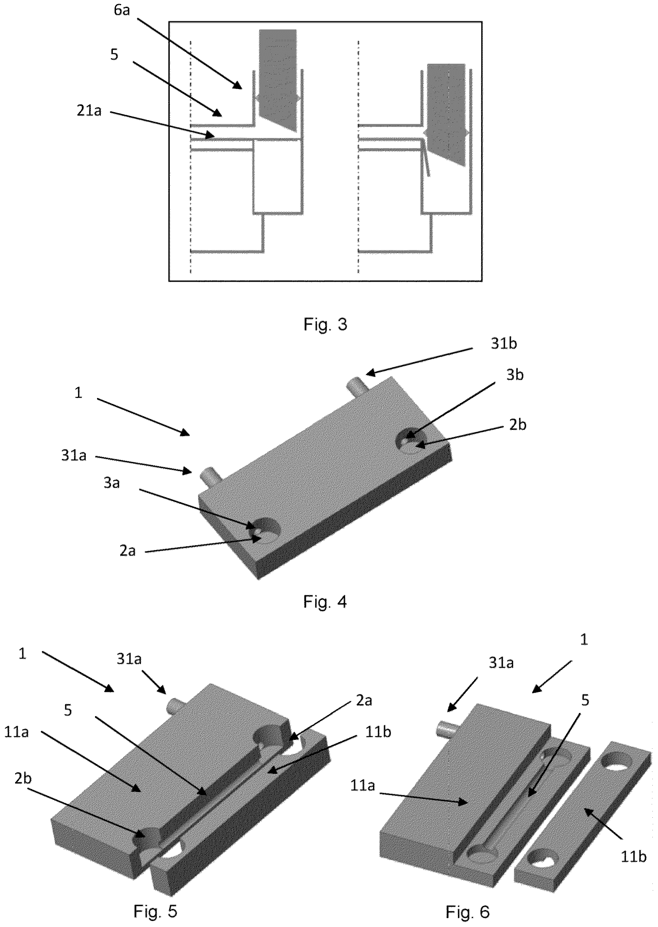

In dieser Reinigungsposition kann aber ein Fluidfluss zwischen den Konnektionstüllen über den Verbindungskanal 5 stattfinden. Verfahren die Anschlusstüllen 6a, 6b in eine in Fig. 2b gezeigte Position weiter, innerhalb der Leitungselemente 2a, 2b weg von der Oberflächenseite des Grundkörpers 1, so gelangen die Stempelelemente schließlich auf eine Position unterhalb der Durchgangslöcher 3a, 3b. In dieser Position kann Fluid aus den Anschlusstüllen 6a, 6b über die Anschlusselemente 31a, 31b in den Konzentratbeutel strömen, wohingegen ein Fluidstrom durch den Verbindungskanal 5 über Seitenflächen der Anschlusstüllen 6a, 6b fluidisch verschlossen ist. Alternativ zeigt Fig. 3 eine Prinzipdarstellung der Reinigungsvorrichtung mit einem Sperrelement 21a. Das Sperrelement 21a ist derart in dem Leitungselement 2a, 2b angeordnet, dass es eine fluidische Verbindung zwischen den Leitungselementen 2a, 2b über den Verbindungskanal 5 zulässt, jedoch eine fluidische Verbindung des Verbindungskanals 5 mit den Anschlusselementen 31a verhindert.

Vorteilhafterweise ist das Sperrelement 21a jeweils als kreisförmiges Sperrelement 21a in Leitungselementen 2a, 2b unterhalb der Unterkante des Verbindungskanals 5 ausgebildet. Werden die Anschlusstüllen 6a, 6b in die zweite Position, das heißt in eine Position in den Leitungselementen 2a, 2b verfahren, in welchen eine fluidische Verbindung mit dem Konzentratbeutel ausgebildet wird, und die Seitenflächen der Anschlusstüllen 6a, 6b damit den Verbindungskanal 5 verschließen, durchstoßen die Anschlusstüllen 6a, 6b die Sperrelemente 21a um in diese Position zu gelangen. Hierfür können die Sperrelemente 21a Sollbruchstellen aufweisen und hierfür beispielsweise in einem Teilabschnitt eine geringere Wandstärke aufweisen oder in einem Teilabschnitt geschwächt ausgebildet sein.

Fig. 4 zeigt eine Prinzipdarstellung der Reinigungsvorrichtung nach einer zweiten Ausführungsform. Die Leitungselemente 2a, 2b erstrecken sich hier senkrecht zu einer Oberfläche des Grundkörpers 1 und sind als Sacklöcher oder Sackbohrungen ausgebildet. An einer von der Oberfläche entfernten Seite, insbesondere unmittelbar an eine Bodenfläche der Leitungselemente 2a, 2b anschließend, sind hier die Durchgangslöcher 3a, 3b ausgebildet. Diese erstrecken sich hier parallel innerhalb des Grundkörpers 1 zu einer Seitenfläche. An diese Durchgangslöcher 3a, 3b anschließend sind die Anschlusselemente 31a, 31b ausgebildet. Diese stehen im Wesentlichen senkrecht von der Oberfläche des Grundkörpers 1 hervor. Werden bei der Verwendung der Reinigungsvorrichtung nach der zweiten Ausführungsform Anschlusstüllen 6a, 6b, wie in Fig. 17 dargestellt, in die Leitungselemente 2a, 2b verfahren, können diese in den Leitungselementen 2a, 2b gespült werden. Dabei strömt aus einer Anschlusstülle 6a, 6b Fluid in eines der Leitungselement 2a, 2b und von einem Leitungselement 2a, 2b über den Verbindungskanal 5 zu dem anderen Leitungselement 2a, bzw. 2b. Die Anschlusselemente 31a, 31b sind während dieses Spülvorgangs, beispielsweise durch ein mit diesen in Verbindung bringbares Verschlusselementen, verschlossen.

Die Anschlusstüllen 6a, 6b werden dabei von dem Fluid umspült. Nach diesem Reinigungsvorgang kann das in den Leitungselementen 2a, 2b verbleibende Fluid über die Durchganglöcher 3a, 3b und sich anschließenden Anschlussleitungen abgesaugt werden. Hierfür ist es vorteilhaft, wenn eine Unterkante der Durchgangslöcher 3a, 3b sich an die Unterseite der Leitungselemente 2a, 2b anschließt. Fig. 5 zeigt eine Prinzipdarstellung der Reinigungsvorrichtung nach einer dritten Ausführungsform. Hierbei ist der Grundkörper 1 aus einem ersten Grundelement 11a, und einem zweiten Grundelement 11b ausgebildet, so dass sich bei einem Verbinden, beispielsweise verschrauben, verkleben oder verschweißen eine Platte ergibt. Der Verbindungskanal 5 entsteht dabei erst nach einem Verbinden der Grundelemente, wobei hier an jedem Grundelement ein Negativ in Form einer im Querschnitt halbkreisförmigen Aussparung gebildet ist.

Diese Aussparungen erstrecken sich entlang ihrer Längsachse im Wesentlichen senkrecht zu den Aussparungen der Leitungselement 2a, 2b, welche aus als im Querschnitt halbkreisförmige Aussparungen ausgebildet sind. Dabei ist nur an dem Leitungselement 2a ein Durchgangloch 3a ausgebildet. Bei einer Verwendung der Reinigungsvorrichtung mit einer Dialysemaschine kann dabei über ein Leitungselement 2a Fluid einströmen, durch den Verbindungskanal 5 zu dem zweiten Leitungselement 2b strömen und von dort über den Verbindungskanal 5 wieder zurückströmen. Am Ende der Reinigung kann über die zuvor, etwa durch ein Ventil, verschlossene Anschlusselement 31a, die nun geöffnet ist, das restliche Fluid entfernt werden.

Fig. 6 zeigt eine Prinzipdarstellung der Reinigungsvorrichtung nach einer vierten Ausführungsform. Auch hier weist der Grundkörper 1 ein erstes und ein zweites Grundelement 11a, 11b auf, welche miteinander verbunden werden, um den Verbindungskanal 5 auszubilden. Auch hier kann die fluidisch dichte Verbindung beispielswiese durch Verkleben, Verschweißen oder Verschrauben erreicht werden. Dabei weist das zweite Grundelement 11b hier zwei Durchgangslöcher oder Durchgangsbohrungen auf, während das erste Grundelement 11a im Durchmesser den Durchgangslöchern entsprechende Sacklöcher aufweist. Zur Ausbildung des Verbindungskanals 5 weist sowohl das erste als auch zweite Grundelement 11a, 11b jeweils im Querschnitt halbkreisförmige, sich zwischen den Durchgangsbohrungen beziehungsweise Sacklöchern erstreckende Ausnehmungen auf.

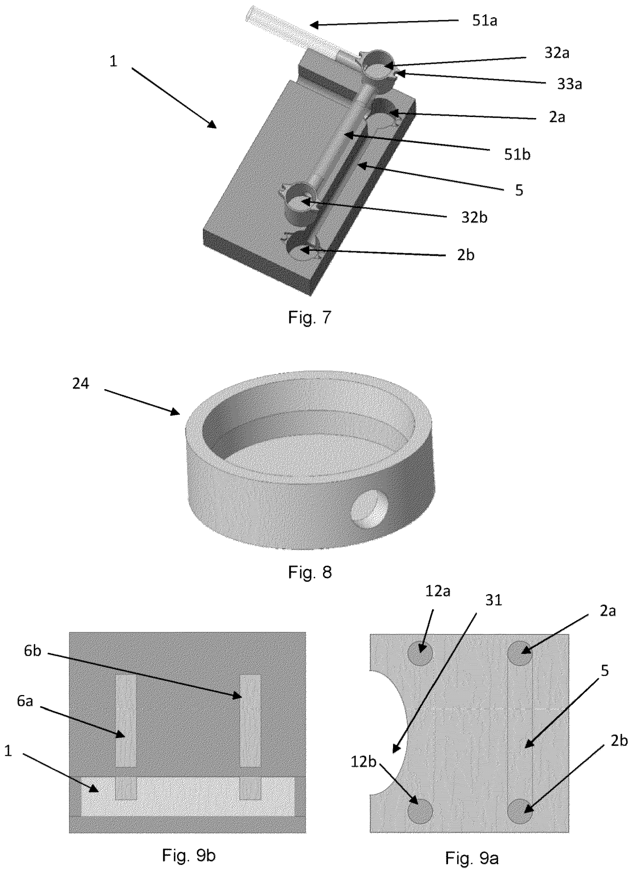

Zudem ist auch in dieser Ausführungsform nur an einem Leitungselement 2a ein Durchgangsloch 3a mit sich daran anschließendem Anschlusselement 31a ausgebildet. Die Reinigung mit in die Leitungselemente 2a, 2b einfahrbaren Anschlusstüllen 6a, 6b einer Dialysemaschine erfolgt entsprechend der Ausführungsform drei. Fig. 7 zeigt eine Prinzipdarstellung der Reinigungsvorrichtung nach einer vierten Ausführungsform. Hierbei weist die Reinigungsvorrichtung weiter eine erste zylinderförmige Aufnahme 32a, hier als Spültopf ausgebildet mit zwei Durchgangsbohrungen, auf. Zusätzlich weist die Reinigungsvorrichtung eine zweite zylinderförmige Aufnahme 32b mit einer Durchgangsbohrung auf. Die Spültöpfe entsprechen in ihrem Außendurchmesser dem Innendurchmesser der Leitungselemente 2a, 2b so dass sie in diese eingefügt werden können.

In der eingefügten Position fluchteten die Durchgangsöffnungen der Aufnahmen 32a, 32b mit dem in dem Grundkörper 1 ausgebildeten Verbindungskanal 5 und dem Durchgangsloch 3a. An Fortsätzen, welche an den Aufnahmen 32a, 32b vorgesehen sind, sind Verbindungselemente 51a, 51b hier als Schläuche ausgebildet, aufgesteckt, beziehungsweise angebracht. Bei dieser Ausführungsform müssen die Aussparungen in dem Grundkörper 1 zur Ausbildung des Verbindungskanals 5, beziehungsweise des Durchgangslochs 3a nicht zur Oberfläche des Grundkörpers 1 verschlossen sein. Vielmehr werden der Verbindungskanal 5 und das Durchgangsloch 3a durch das Verbindungselement 51a, 51b derart ausgebildet, dass diese zur Oberfläche verschlossen sind. In der Grundplatte können wie in Fig. 7 dargestellt, Gewindebohrungen angebracht sein, so dass die Aufnahmen, welche seitliche Laschen 33a aufweisen, in dem Grundkörper 1 verschraubt werden können. Alternativ können die Aufnahmen durch eine Presspassung mit den Leitungselementen 2a, 2b in dem Grundkörper 1 befestigt oder in diesem verklebt werden.

Fig. 8 zeigt eine Prinzipdarstellung einer Reinigungsschale für eine Reinigungsvorrichtung, insbesondere zur Einbringung in die Leitungselemente 2a, 2b. Um eine fluidische Verbindung herstellen zu können, sind in den Reinigungsschalen jeweils Durchgangsbohrungen, in der hier gezeigten Dichtschale 24 eine Durchgangsbohrung, ausgebildet. Fig. 9a zeigt eine schematische Darstellung einer Reinigungsvorrichtung nach einer sechsten Ausführungsform. Hierbei kann an der Reinigungsvorrichtung ein Konzentratbeutel in der Aufnahme 31 angebracht werden. Der Konzentratbeutel kann hierfür beispielsweise mit Ausnehmungen 12a, 12b in fluidischer Verbindung stehen oder in diese eingepresst werden. Die Ausnehmungen 12a, 12b können in der Reinigungsvorrichtung ausgebildet sein, und eine fluidische Verbindung zwischen einem Konzentratbeutel und den Konnektionstüllen 6a, 6b zulassen. Die Reinigungsvorrichtung weist weiter zwei Leitungselemente 2a, 2b auf, welche über einen Verbindungskanal 5 verbunden sind. Dabei kann die Reinigungsvorrichtung derart verschiebbar in einer Dialysemaschine angeordnet sein, dass Anschlusstüllen 6a, 6b der Dialysemaschine, welche vertikal verschiebbar angeordnet sind, in die Leitungselemente 2a, 2b zur Reinigung der Anschlusstüllen 6a, 6b einfahren können, und nach einem horizontalen Verschieben der Reinigungsvorrichtung in die Ausnehmungen 12a, 12b einfahren können, umso mit dem Konzentratbeutel fluidisch verbunden zu sein.

Hängt ein Konzentratbeutel in der dafür vorgesehenen Aufnahme 31, verfährt die Reinigungsvorrichtung horizontal in ein Geräteinneres der Dialysemaschine und positioniert so den Konzentratbeuteladapter unter den Konnektionstüllen. Diese Konnektionstüllen verfahren vertikal nach unten in Öffnungen des Konzentratbeutels und konnektieren so den Beutel für den Dialysebetrieb. Sobald die Therapie beendet und der Konzentratbeutel entleert ist, bewegen sich die Konnektionstüllen vertikal nach oben aus dem Konzentratbeuteladapter heraus. Anschließend fährt die Reinigungsvorrichtung horizontal aus dem Dialysegerät teilweise heraus in die Reinigungsposition. In der Reinigungsposition verfahren die Konnektionstüllen wieder in die Leitungselemente 2a, 2b. Der Konzentratbeutel hängt in dieser Stellung der Reinigungseinheit vor der Dialysemaschine und kann entnommen werden. Nach der anschließenden Oberflächenreinigung wird ein neuer Beutel eingehängt.

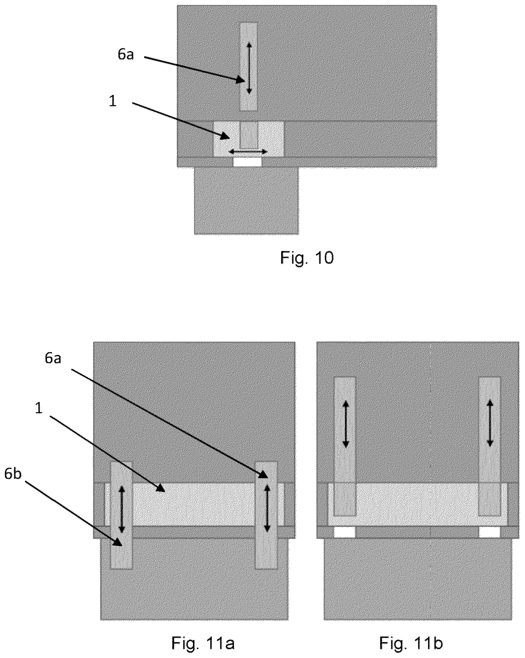

Fig. 10 zeigt eine weitere Prinzipdarstellung für eine Verwendung einer Reinigungsvorrichtung an einer Dialysemaschine. Der Konzentratbeutel wird vom Pflegepersonal an einem Konnektorgehäuse, in welchem die Konnektionstüllen 6a, 6b angeordnet sind, der Dialysemaschine in eine Aufnahme eingesetzt. Die Aufnahme ist so konzipiert, dass die Verbindungsöffnungen im Konzentratbeutel genau unterhalb der Anschlusstüllen 6a, 6b liegen. Nach dem Beenden der Reinigung fahren die Anschlusstüllen 6a, 6b vertikal nach oben aus der Reinigungsvorrichtung. Durch Öffnungen in dem Konnektorgehäuse können die Anschlusstüllen 6a, 6b bei ihrer vertikalen Abwärtsbewegung den Konzentratbeutel an einen Dialysatkreislauf anschließen. Nach der Therapie und dem Entleeren des Konzentratbeutels kehren die Anschlusstüllen 6a, 6b in die obere Endlage zurück. Die Reinigungsvorrichtung fährt anschließend horizontal bis unter die Anschlusstüllen 6a, 6b, sodass diese für die Reinigung in die Leitungselemente 2a, 2b fahren können. Der Konzentratbeutel kann nun entnommen und die Oberflächendesinfektion durchgeführt werden. Anschließend wird ein neuer Konzentratbeutel in die Aufnahme eingesetzt.

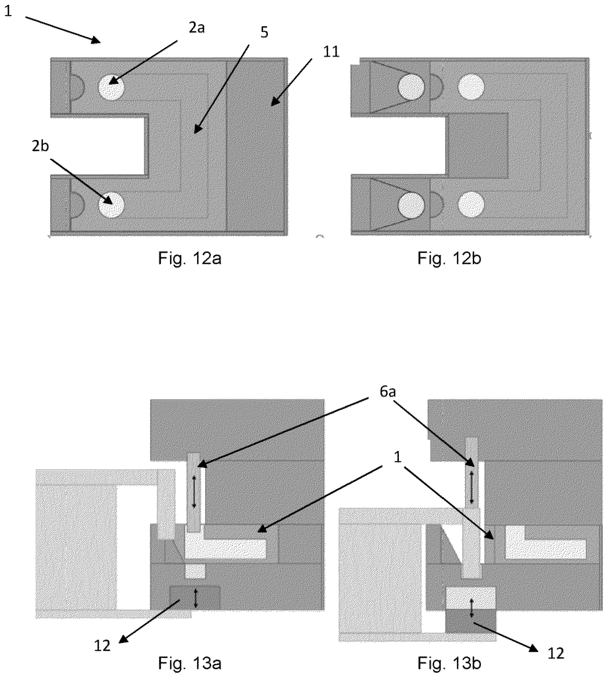

Fig. 11 a) und b) zeigen die zuvor beschriebene Anordnung aus einer Vorderansicht mit einer Konnektionsstellung Fig. 11a und einer Reinigungsstellung Fig. 11b. Fig. 12 zeigt eine Prinzipdarstellung der Reinigungsvorrichtung nach einer achten Ausführungsform. Bei dieser Ausführungsform weist die Reinigungsvorrichtung eine 11 Aufnahmeeinheit zur Aufnahme eines Konzentratbeutels auf, wobei die Reinigungsvorrichtung zu der Aufnahmeeinheit beweglich, insbesondere verschiebbar, ausgebildet ist. Der Verbindungskanal 5 verläuft zwischen den Leitungselementen 2a, 2b, in einem Mittelabschnitt wobei der Verbindungskanal U-förmig ausgebildet ist, so dass zwischen den Leitungselementen 2a, 2b ein Freiraum ausgebildet ist. Durch ein Stellelement, beispielsweise ein Feder, kann eine bestimmte Relativposition zwischen Reinigungsvorrichtung und Aufnahmeeinheit sichergestellt werden, wobei die Feder an der Dialysemaschine selbst angebracht sein kann, so dass die Reinigungsvorrichtung immer eine Kraft erfährt, welche sie in die Reinigungsstellung bringt.

Die Aufnahmeeinheit weist Aufnahmen, beispielsweise Aufnahmebohrungen, für einen Konzentratbeutel auf, so dass der Konzentratbeutel derart positioniert wird, dass die Anschlusstüllen 6a, 6b der Dialysemaschine sicher in diesen verfahren werden können. Hierfür sind bei dieser Ausführungsform in der Reinigungsstellung die Aufnahmebohrungen mit den Leitungselementen 2a, 2b fluchtend angeordnet, während die Reinigungsvorrichtung bei einem sich in der Aufnahmeeinheit befindendem Konzentratbeutel, in der Konnektionsstellung derart verschoben, dass dieser unter den Konnektionstüllen angeordnet ist. Nachfolgend wird ein Ablauf eines Reinigungsverfahrens beschrieben. Dabei ist die Startposition der Reinigungsvorrichtung die Reinigungsstellung, wie in Fig. 12 a, b gezeigt. In dieser Position kann ein Konzentratbeutel in die Aufnahmeeinheit eingehängt werden. Die Positionierung kann durch an der Aufnahmeeinheit vorgesehene Schrägen, sowie in der Reinigungsvorrichtung ausgebildete Aussparungen, beispielsweise halbkreisförmige Rillen, erleichtert werden. Mit anderen Worten kann das Pflegepersonal, während sich die Reinigungsvorrichtung in einer vorderen Position, wie in Fig. 12 gezeigt, befindet, bereits einen neuen Konzentratbeutel an der Aufnahmeeinheit einhängen.

Ein in Fig. 13 a und b gezeigtes Einpresselement 12 kann weiter an der Unterseite der Aufnahmeeinheit, beziehungsweise des Gehäuses 9, ausgebildet. Das Einpresselement kann über eine vorgespannte Feder in der Aufnahmeeinheit angeordnet sein, dass der Konzentratbeutel in eine Stellung unterhalb der Anschlusstüllen 6a, 6b gedrückt wird. Hierfür wird das Einpresselement erst durch ein Anbringen eines Konzentratbeutels in die Aufnahmeeinheit eingedrückt. Sobald die Anschlusstüllen 6a, 6b aus den Leitungselementen 2a, 2b verfahren sind, und die Leitungselemente 2a, 2b damit verschiebbar ist, wird das Einpresselement durch die Federkraft aus der Aufnahmeeinheit gedrückt und verschiebt den Konzentratbeutel unter die Anschlusstüllen 6a, 6b. Mit anderen Worten fahren nach der Reinigung des Dialysatkreislaufs die beiden Anschlusstüllen 6a, 6b vertikal nach oben aus den Leitungselementen 2a, 2b heraus. Im Anschluss daran fährt das Einpresselement 12 auf der Unterseite der Aufnahmeeinheit heraus und drückt so den Konzentratbeutel entlang der Schräge in die Aufnahmebohrungen.

Die Bewegung entlang der Schräge beinhaltet neben der vertikalen auch eine horizontale Richtung, wodurch die Reinigungsvorrichtung horizontal verschoben wird, so dass die Aufnahmebohrungen frei werden. Um den Konzentratbeutel an den Dialysatkreislauf anzuschließen, fahren die Anschlusstüllen 6a, 6b vertikal nach unten und konnektieren den Konzentratbeutel an den entsprechenden Öffnungen. Nach der Therapie wird der Konzentratbeutel entleert, die Anschlusstüllen 6a, 6b bewegen sich aus dem Konzentratbeutel und das Einpressstück 12 fährt in die Aufnahmeeinheit zurück. Anschließend kann das Pflegepersonal den Konzentratbeutel entnehmen. Durch die Entnahme des Konzentratbeutels stellt sich die Reinigungsvorrichtung zurück in die Reinigungsstellung, sodass die Anschlusstüllen 6a, 6b wieder vertikal nach unten in die Reinigungsstellung fahren können. Während der Dialysatkreislaufreinigung erfolgt die Oberflächendesinfektion, sowie das Einhängen eines neuen Konzentratbeutels durch das Pflegepersonal.

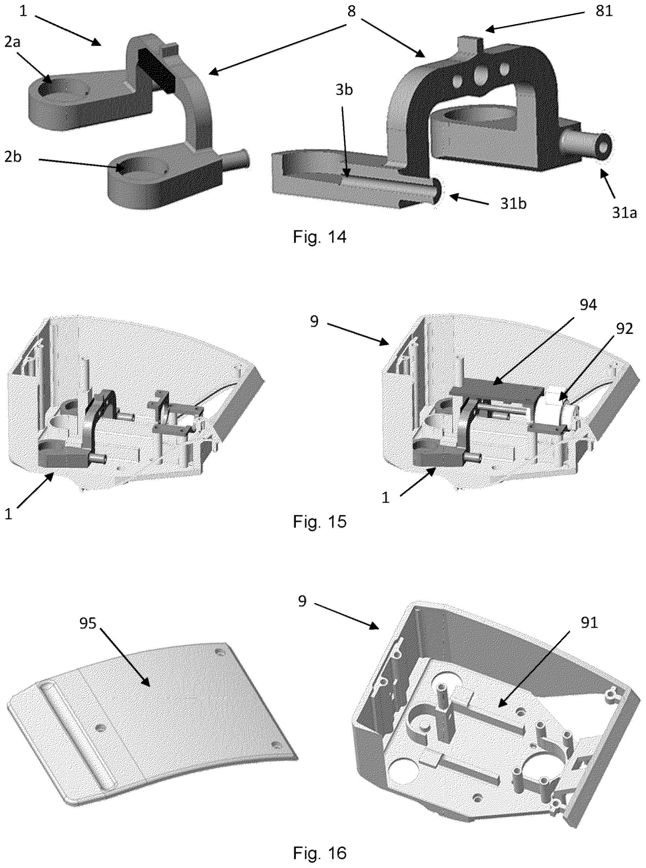

Fig. 14 zeigt eine Prinzipdarstellung einer Verwendung der Reinigungsvorrichtung nach einer neunten Ausführungsform. Dabei weist der Grundkörper 1 zwei armähnliche Elemente auf, in welchen die Leitungselemente 2a, 2b ausgebildet sind. Zwischen diesen armähnlichen Elementen ist ein sich in entgegengesetzter Richtung zu der Richtung, in welcher sich die Leitungselemente 2a, 2b in dem Grundkörper 1 erstrecken, bogenförmiges Mittelelement 8 ausgebildet. Jeder der zwei Leitungselemente 2a, 2b ist mit einem Durchgangsloch 3a verbunden an welchen sich je ein Anschlusselement 31a, 31b anschließt. In dem Mittelelement 8 kann weiter eine Bohrung zur Aufnahme einer Gewindemutter ausgebildet sein. Alternativ kann die Gewindemutter direkt in das Mittelelement 8 geschnitten ausgebildet sein.

Zudem kann an dem Mittelelement 8 ein Positionselement 81 ausgebildet sein, welches mit einem der Dialysemaschine zugeordneten Sensor wechselwirkt, und beispielsweise als Vorsprung 81 von dem Mittelelement 8 eine Lichtschranke auslösen kann. Fig. 15 zeigt eine Prinzipdarstellung eines Reinigungssystems. Hier ist eine Reinigungsvorrichtung nach der zuvor genannten Ausführungsform in einem Gehäuse 9 angeordnet. Zur Führung der Reinigungsvorrichtung können in dem Gehäuse 9 Führungsschienen 91 ausgebildet sein. Die Führungsschienen 91 können zwei symmetrisch angeordnete Balken am Boden des Gehäuses 9 sein. Um ein Verkippen der Reinigungsvorrichtung zu verhindern, können sich an einem Ende der Balken horizontale Flächen befinden, die immer einen Bereich der Reinigungsvorrichtung überdecken.

An den Anschlusselementen 31a, 31b können zur Regelung eines Fluidstroms nicht dargestellte Sperrelemente, beispielsweise Ventile, ausgebildet sein. Zur Bewegung der Reinigungsvorrichtung ist ein Antriebselement in dem Gehäuse 9 angeordnet. Zur Erfassen der Bewegung der Reinigungsvorrichtung ist eine Platine derart in dem Gehäuse 9 angeordnet, dass der Vorsprung 81 am Mittelement 8 mit an der Platine 94 versehenen Gabellichtschranken wechselwirken kann. Das Antriebselement in dieser Ausführungsform ist ein Elektromotor 92, welcher eine in die Gewindemutter am Mittelelement 8 eingreifende Gewindespindel antreibt. Das Gehäuse 9 kann ferner einen Deckel 95 aufweisen, welcher nach der Montage der Elemente auf dem Gehäuse 9 verschraubt werden kann.

Ferner kann das Gehäuse 9 einen Deckel 95, ein Dichtelement zwischen dem Dialysegerät und dem Gehäuse 9, sowie einen Befestigungsadapter aufweisen. Zur erleichterten Positionierung des Deckels 95 auf dem Gehäuse 9 befinden sich an einer Unterseite, an einer Vorderseite, sowie an zwei weiteren Seiten Positionierhilfen in Form von Anschlägen, wie in Fig. 16 gezeigt.

Nach dem Einsetzen des Deckels 95 wird dieser an drei Stellen mit dem Gehäuse 9 verschraubt. Auf die eingeschraubten Schraubenköpfe wird ein Gummistopfen in der Farbe des Deckels 95 gesteckt. Sie sollen die Bohrungen schließen, sodass der Deckel 95 eine ebene Fläche besitzt. Die Rille, wie in Fig. 16 gezeigt, auf der oberen Seite des Deckels 95 dient der Aufnahme des Konzentratbeutels am Gehäuse 9. Aufgrund der Schwächung der Wandstärke des Deckels 95 an dieser Stelle wird die Unterseite dort mit Material verstärkt. Um das Einhängen des Konzentratbeutels zu erleichtern, fällt die Fläche zwischen der Rille bis zur Vorderkante hin ab. Das Gehäuse 9 kann auch seinen Durchmesser zwischen den Seitenflächen nach vorne verringern, so dass ein Konzentratbeutel leicht über das Gehäuse 9 aufgeschoben werden kann.

## Patentansprüche

1. Reinigungsvorrichtung für eine Blutbehandlungsvorrichtung, insbesondere eine Dialysemaschine, aufweisend
einen Grundkörper (1),
ein erstes und ein zweites Leitungselement (2a, 2b), welche sich in dem Grundkörper (1) von einer ersten Oberfläche des Grundkörpers (1) erstrecken,
ein erstes Durchgangsloch (3a), wobei sich das erste Durchgangsloch (3a) an einem von der ersten Oberfläche des Grundkörpers (1) beabstandeten ersten Stelle des ersten Leitungselements (2a) zu einer von der ersten Oberfläche des Grundkörpers (1) verschiedenen Oberfläche des Grundkörpers (1) erstreckt,
, **dadurch gekennzeichnet, dass**
ein Verbindungskanal (5) zwischen dem ersten und dem zweiten Leitungselement (2a, 2b) an einer von der Oberfläche des Grundkörpers (1) entfernten zweiten Stelle der Leitungselemente (2a, 2b) ausgebildet ist.

2. Reinigungsvorrichtung nach Anspruch 1, wobei
ein erstes Anschlusselement (31a), welches sich von dem Grundkörper (1) entlang des ersten Durchgangslochs (3a) von der ersten Seitenfläche des Grundkörpers (1) erstreckt, so dass sich das erste Durchgangsloch (3a) von der ersten Seitenfläche des Grundkörpers (1) weiter durch das erste Anschlusselement (31a) erstreckt.

3. Reinigungsvorrichtung nach Anspruch 1 oder 2, wobei
sich das erste Durchgangsloch (3a) zu einer ersten Seitenfläche des Grundkörpers (1) erstreckt,
das zweite Leitungselement (2b) an einer von der ersten Oberfläche des Grundkörpers (1) entfernten dritten Stelle ein zweites Durchgangsloch (3b) zu der von der ersten Oberfläche des Grundkörpers (1) verschiedenen ersten Seitenfläche des Grundkörpers (1) aufweist.

4. Reinigungsvorrichtung nach Ansprüchen 1 bis 3, wobei
in dem ersten und zweiten Leitungselement (2a, 2b) zumindest ein Sperrelement (21a) angebracht ist, so dass keine fluidische Verbindung zwischen dem Verbindungskanal (5) mit dem ersten und zweiten Durchgangsloch (3a, 3b) besteht, und vorzugsweise das Sperrelement (21a) Sollbruchstellen aufweist.

5. Reinigungsvorrichtung nach einem der vorangegangenen Ansprüche,
wobei der Grundkörper (1) aus einem ersten Grundelement (11a) und einem zweiten Grundelement (11b) ausgebildet ist, wobei jeweils das erste und zweite Grundelement (11a, 11b) eine im Querschnitt halbkreisförmige Rille zur Ausbildung des Verbindungskanals (5) aufweisen, und das erste und zweite Grundelement (11a, 11b) jeweils zwei im Querschnitt halbkreisförmige Aussparungen aufweisen zur Ausbildung von jeweils dem ersten und zweiten Leitungselement (2a, 2b) bei Verbindung des ersten und zweiten Grundelements (11a, 11b).

6. Reinigungsvorrichtung nach einem der vorangegangenen Ansprüche,
wobei der Grundkörper (1) aus einem ersten Grundelement (11a) und einem zweiten Grundelement (11b) ausgebildet ist, und das erste Grundelement (11a) zwei Sackbohrungen und das zweite Grundelement (11b) zwei den im Durchmesser der Sackbohrungen entsprechende Durchgangsbohrungen zur Ausbildung des ersten und zweiten Leitungselements (2a, 2b) aufweisen, sowie das erste Grundelement (11a) und zweite Grundelement (11b) eine im Querschnitt halbkreisförmige Rille zur Ausbildung des Verbindungskanals (5) aufweisen.

7. Reinigungsvorrichtung nach einem der vorangegangenen Ansprüche, weiter aufweisend
eine erste zylinderförmige Aufnahme (32a) mit zumindest einer, vorzugsweise zwei Durchgangsöffnungen und/oder eine zweite zylinderförmige Aufnahme (32b) mit einer Durchgangsöffnung, wobei an den Durchgangsöffnungen jeweils ein zylinderförmiger Fortsatz vorgesehen ist, und
zumindest ein, vorzugsweise zwei, röhrenförmige Verbindungselemente (51a, 51b), welche auf den Fortsätzen anbringbar sind und in den Verbindungskanal (5) und/oder dem Durchgangsloch (3a, 3b) derart eingebracht werden können, dass sie die in den Leitungselementen (2a, 2b) aufgenommenen Aufnahmen (32a, 32b) verbinden, wobei
das erste Durchgangsloch (3a, 3b) sowie der Verbindungskanal (5) zur ersten Oberfläche geöffnet sind, so dass jeweils die zylinderförmigen Aufnahmen (32a, 32b) sowie die röhrenförmigen Verbindungselemente (51a, 51b) in den Grundkörper (1) einlegbar sind.

8. Reinigungsvorrichtung nach einem der vorangehenden Ansprüche,
wobei ein zwischen dem ersten und zweiten Leitungselement (2a, 2b) liegendes Mittelelement (8) bogenförmig ausgebildet ist, und sich von der ersten Oberfläche, vorzugsweise senkrecht, wegerstreckt.

9. Reinigungsvorrichtung nach einem der vorangehenden Ansprüche, weiter aufweisend
zumindest eine Dichtschale (24) in dem ersten und/oder zweiten Leitungselement (2a, 2b), wobei die Dichtschale (24) zylinderförmig ist, und zumindest eine Seitendurchgangsöffnung aufweist, die in ihrem Durchmesser dem Verbindungskanal (5) und/oder dem Durchgangsloch (3a, 3b) entspricht, und an der Mantelfläche der Dichtschale (24) ausgebildet ist.

10. Reinigungssystem für eine Blutbehandlungsvorrichtung, insbesondere eine Dialysemaschine, aufweisend,
ein Gehäuse (9) zur Anbindung an eine Blutbehandlungsvorrichtung, insbesondere eine Dialysemaschine,
eine Reinigungsvorrichtung nach einem der Ansprüche 1 bis 9, die in dem Gehäuse (9) bewegbar angeordnet ist,
ein erstes Verschlusselement mit welchem eine Fluidströmung aus dem ersten Leitungselement (2a) über das erste Anschlusselement (31a) verhinderbar ist, und/oder ein zweites Verschlusselement mit welchem eine Fluidströmung aus dem zweiten Leitungselement (2b) über das zweite Anschlusselement (31b) verhinderbar ist,
ein Antriebselement zum Bewegen der Reinigungsvorrichtung,
einen, vorzugsweise in dem Gehäuse (9), angebrachten Sensor zum Erfassen einer Bewegung der Reinigungsvorrichtung.

11. Reinigungssystem nach Anspruch 10, wobei
der Sensor eine Lichtschranke auf einer oberhalb der Reinigungsvorrichtung angebrachten Platine (94) ist.

12. Reinigungssystem nach Anspruch 10 oder 11, weiter aufweisend
zumindest eine in dem Gehäuse (9), vorzugsweise integral, ausgebildete Führungsschiene (91), auf welcher die Reinigungsvorrichtung verschiebbar ist, und/oder
das erste Antriebselement eine Spindelmutter und einen Elektromotor mit einer Gewindespindel aufweist.

13. Reinigungssystem nach einem der Ansprüche 10 bis 12, wobei
die Gewindemutter an dem, vorzugsweise bogenförmig ausgebildeten, Mittelelement (8) der Reinigungsvorrichtung ausgebildet ist und ein anderes Material als der Grundkörper (1) aufweist, und/oder
das bogenförmig ausgebildete Mittelelement (8) einen in Richtung der Lichtschranke ausgebildeten Vorsprung (81) aufweist, so dass bei einem Bewegen, insbesondere einem Verschieben, der Reinigungsvorrichtung ein Auslösen der Lichtschranke erreichbar ist.

14. Verwendung eines Reinigungssystems nach einem der Ansprüche 10 bis 13 bei einer Blutbehandlungsvorrichtung, insbesondere einer Dialysemaschine.

15. Verwendung einer Reinigungsvorrichtung nach einem der Ansprüche 1 bis 9 bei einer Blutbehandlungsvorrichtung, insbesondere einer Dialysemaschine.

## Claims

1. Cleaning device for a blood treatment device, in particular a dialysis machine, having
a base body (1),
a first and a second line element (2a, 2b), which extend in the base body (1) from a first surface of the base body (1),
a first through-hole (3a), wherein the first through-hole (3a) extends, at a first point of the first line element (2a) spaced apart from the first surface of the base body (1), to a surface of the base body (1) different from the first surface of the base body (1),
**characterized in that**
a connecting duct (5) between the first and the second line element (2a, 2b) is formed at a second point of the line elements (2a, 2b) that is distant from the surface of the base body (1).

2. Cleaning device according to Claim 1, wherein
a first connection element (31a), which extends from the base body (1) along the first through-hole (3a) from the first side face of the base body (1), such that the first through-hole (3a) extends from the first side face of the base body (1) further through the first connection element (31a).

3. Cleaning device according to Claim 1 or 2, wherein
the first through-hole (3a) extends to a first side face of the base body (1),
the second line element (2b), at a third point spaced apart from the first surface of the base body (1), has a second through-hole (3b) to the first side face of the base body (1) different from the first surface of the base body (1).

4. Cleaning device according to Claims 1 to 3, wherein
at least one blocking element (21a) is mounted in the first and second line element (2a, 2b), such that there is no fluidic connection between the connecting duct (5) to the first and second through-hole (3a, 3b), and the blocking element (21a) preferably has predetermined breaking points.

5. Cleaning device according to one of the preceding claims,
wherein the base body (1) is formed of a first base element (11a) and a second base element (11b), wherein the first and second base element (11a, 11b) each have a groove, which is semicircular in cross section, for forming the connecting duct (5), and the first and second base element (11a, 11b) each have two recesses, which are semicircular in cross section, for respectively forming the first and second line element (2a, 2b) when the first and second base element (11a, 11b) are connected.

6. Cleaning device according to one of the preceding claims,
wherein the base body (1) is formed of a first base element (11a) and a second base element (11b), and the first base element (11a) has two blind bores, and the second base element (11b) has two through-bores, corresponding in diameter to the blind bores, for forming the first and second line element (2a, 2b), and the first base element (11a) and second base element (11b) have a groove, which is semicircular in cross section, for forming the connecting duct (5).

7. Cleaning device according to one of the preceding claims, further having
a first cylindrical receptacle (32a) comprising at least one through-opening, preferably two through-openings, and/or a second cylindrical receptacle (32b) comprising one through-opening, wherein a cylindrical extension is provided on each of the through-openings, and
at least one tubular connecting element, preferably two tubular connecting elements (51a, 51b), which can be mounted on the extensions and which can be introduced into the connecting duct (5) and/or the through-hole (3a, 3b) in such a way that they connect the receptacles (32a, 32b) received in the line elements (2a, 2b), wherein
the first through-hole (3a, 3b) and the connecting duct (5) are open towards the first surface, so that the cylindrical receptacles (32a, 32b) and the tubular connecting elements (51a, 51b) can each be placed into the base body (1).

8. Cleaning device according to one of the preceding claims,
wherein a middle element (8) located between the first and second line element (2a, 2b) is arc-shaped and extends away, preferably perpendicularly, from the first surface.

9. Cleaning device according to one of the preceding claims, further having
at least one sealing shell (24) in the first and/or second line element (2a, 2b), wherein the sealing shell (24) is cylindrical and has at least one lateral through-opening, which corresponds in its diameter to the connecting duct (5) and/or to the through-hole (3a, 3b) and is formed on the jacket surface of the sealing shell (24) .

10. Cleaning system for a blood treatment device, in particular a dialysis machine, having
a housing (9) for connection to a blood treatment device, in particular a dialysis machine,
a cleaning device according to one of Claims 1 to 9, which is arranged movably in the housing,
a first closure element, by which a flow of fluid from the first line element (2a) via the first connection element (31a) can be prevented, and/or a second closure element, by which a flow of fluid from the second line element (2b) via the second connection element (31b) can be prevented,
a drive element for moving the cleaning device,
a sensor, preferably mounted in the housing (9), for detecting a movement of the cleaning device.

11. Cleaning system according to Claim 10, wherein
the sensor is a light barrier on a printed circuit board (94) mounted above the cleaning device.

12. Cleaning system according to Claim 10 or 11, further having
at least one guide rail (91), which is formed, preferably integrally, in the housing (9) and on which the cleaning device is movable, and/or
the first drive element has a spindle nut and an electric motor with a threaded spindle.

13. Cleaning system according to one of Claims 10 to 12, wherein
the threaded nut is formed on the preferably arc-shaped middle element (8) of the cleaning device and has a material different than the base body (1), and/or
the arc-shaped middle element (8) has a protrusion (81), which is formed in the direction of the light barrier, such that triggering of the light barrier can be achieved upon movement, in particular shifting, of the cleaning device.

14. Use of a cleaning system according to one of Claims 10 to 13 in a blood treatment device, in particular in a dialysis machine.

15. Use of a cleaning device according to one of Claims 1 to 9 in a blood treatment device, in particular in a dialysis machine.

## Revendications

1. Dispositif de nettoyage d'un dispositif de traitement du sang, notamment d'une machine de dialyse, ledit dispositif de nettoyage comportant
un corps de base (1),
des premier et deuxième éléments de conduite (2a, 2b) qui s'étendent dans le corps de base (1) depuis une première surface du corps de base (1),
un premier trou traversant (3a), le premier trou traversant (3a) s'étendant, à un premier emplacement du premier élément de conduite (2a) distant de la première surface du corps de base (1), jusqu'à une surface du corps de base (1) qui est différente de la première surface du corps de base (1),
**caractérisé en ce que**
un conduit de liaison (5) est formé entre les premier et deuxième éléments de conduite (2a, 2b) à un deuxième emplacement des éléments de conduite (2a, 2b) éloigné de la surface du corps de base (1).

2. Dispositif de nettoyage selon la revendication 1, un premier élément de raccordement (31a) s'étend du corps de base (1) le long du premier trou traversant (3a) depuis la première surface latérale du corps de base (1), de sorte que le premier trou traversant (3a) s'étend davantage à travers le premier élément de raccordement (31a) depuis la première surface latérale du corps de base (1).

3. Dispositif de nettoyage selon la revendication 1 ou 2,
le premier trou traversant (3a) s'étendant jusqu'à une première surface latérale du corps de base (1),
le deuxième élément de conduite (2b) comportant un deuxième trou traversant (3b) à un troisième emplacement éloigné de la première surface du corps de base (1) jusqu'à la première surface latérale du corps de base (1), qui est différente de la première surface du corps de base (1).

4. Dispositif de nettoyage selon les revendications 1 à 3,
au moins un élément de blocage (21a) étant monté dans les premier et deuxième éléments de conduite (2a, 2b) de sorte qu'aucune liaison fluidique n'existe entre le conduit de liaison (5) et les premier et deuxième trous traversants (3a, 3b), et de préférence l'élément de blocage (21a) présentant des points de rupture.

5. Dispositif de nettoyage selon l'une des revendications précédentes,
le corps de base (1) étant formé à partir d'un premier élément de base (11a) et d'un deuxième élément de base (11b), les premier et deuxième éléments de base (11a, 11b) comportant chacun une rainure à section semi-circulaire afin de former le conduit de liaison (5), et les premier et deuxième éléments de base (11a, 11b) comportant chacun deux évidements à section semi-circulaire afin de former chacun les premier et deuxième éléments de conduite (2a, 2b) lors de la liaison des premier et deuxième éléments de base (11a, 11b) .

6. Dispositif de nettoyage selon l'une des revendications précédentes,
le corps de base (1) étant formé d'un premier élément de base (11a) et d'un deuxième élément de base (11b), et le premier élément de base (11a) comportant deux trous borgnes et le deuxième élément de base (11b) comportant deux trous traversants correspondant au diamètre des trous borgnes afin de former les premier et deuxième éléments de conduite (2a, 2b), et le premier élément de base (11a) et le deuxième élément de base (11b) comportant une rainure à section semi-circulaire afin de former le conduit de liaison (5).

7. Dispositif de nettoyage selon l'une des revendications précédentes, comprenant en outre
un premier réceptacle cylindrique (32a) pourvu d'au moins une ouverture traversante, de préférence deux ouvertures traversantes et/ou un deuxième réceptacle cylindrique (32b) pourvu d'une ouverture traversante étant prévus, une extension cylindrique étant prévue au niveau de chaque ouverture traversante, et
au moins un élément de liaison tubulaire, de préférence deux éléments de liaison tubulaires (51a, 51b), qui peuvent être montés sur les extensions et qui peuvent être introduits dans le conduit de liaison (5) et/ou le trou traversant (3a, 3b) de manière à relier les réceptacles (32a, 32b) reçus dans les éléments de conduite (2a, 2b),
le premier trou traversant (3a, 3b) et le conduit de liaison (5) étant ouverts sur la première surface de sorte que les réceptacles cylindriques (32a, 32b) et les éléments de liaison tubulaires (51a, 51b) puissent être insérés dans le corps de base (1).

8. Dispositif de nettoyage selon l'une des revendications précédentes,
un élément central (8) situé entre les premier et deuxième éléments de conduite (2a, 2b) étant en forme d'arc et s'étendant à l'opposé de la première surface, de préférence perpendiculairement.

9. Dispositif de nettoyage selon l'une des revendications précédentes, comportant en outre
au moins une coque d'étanchéité (24) dans le premier et/ou le deuxième élément de conduite (2a, 2b),
la coque d'étanchéité (24) étant cylindrique et comportant au moins une ouverture traversante latérale dont le diamètre correspond au conduit de liaison (5) et/ou au trou traversant (3a, 3b), et étant formée sur la surface latérale de la coque d'étanchéité (24).

10. Système de nettoyage d'un dispositif de traitement du sang, notamment d'une machine de dialyse, ledit système de nettoyage comportant,
un boîtier (9) de raccordement à un dispositif de traitement du sang, notamment une machine de dialyse,
un dispositif de nettoyage selon l'une des revendications 1 à 9, disposé de manière mobile dans le boîtier (9),
un premier élément de fermeture qui permet d'empêcher un écoulement de fluide depuis le premier élément de conduite (2a) via le premier élément de raccordement (31a), et/ou un deuxième élément de fermeture qui permet d'empêcher un écoulement de fluide depuis le deuxième élément de conduite (2b) via le deuxième élément de raccordement (31b),
un élément d'entraînement destiné à déplacer le dispositif de nettoyage,
un capteur, de préférence monté dans le boîtier (9), destiné à détecter un mouvement du dispositif de nettoyage.

11. Système de nettoyage selon la revendication 10,
le capteur étant une barrière lumineuse sur une carte (94) montée au-dessus du dispositif de nettoyage.

12. Système de nettoyage selon la revendication 10 ou 11, comportant en outre
au moins un rail de guidage (91) qui est formé de préférence d'un seul tenant dans le boîtier (9) et sur lequel le dispositif de nettoyage peut coulisser, et/ou
le premier élément d'entraînement comportant un écrou de broche et un moteur électrique pourvu d'une broche filetée.

13. Système de nettoyage selon l'une des revendications 10 à 12,
l'écrou fileté étant formé sur l'élément central (8), de préférence en forme d'arc, du dispositif de nettoyage et comportant un matériau différent de celui du corps de base (1), et/ou
l'élément central (8) en forme d'arc comportant une saillie (81) formée en direction de la barrière lumineuse, de manière à pouvoir déclencher la barrière lumineuse lors d'un déplacement, en particulier d'un coulissement, du dispositif de nettoyage.

14. Utilisation d'un système de nettoyage selon l'une des revendications 10 à 13 dans un dispositif de traitement du sang, notamment une machine de dialyse.

15. Utilisation d'un dispositif de nettoyage selon l'une des revendications 1 à 9 dans un dispositif de traitement du sang, notamment une machine de dialyse.
